# EUROPÄISCHE PATENTSCHRIFT

(11) **EP 1 048 292 B1**
(45) Veröffentlichungstag und Bekanntmachung des Hinweises auf die Patenterteilung: **12.12.2007**
(21) Anmeldenummer: 00107918.5
(22) Anmeldetag: 13.04.2000
(51) Int. Cl.: A61K 8/368, A61K 8/60, A61Q 19/00, A61P 17/10

(54) **Stabile Wirkstoffkombinationen mit einem Gehalt an grenzflächenaktiven Glucosederivaten und Salicylsäure**
Stable combination of active agents containing surface-active gucose derivatives and salicylic acid
Combinaisons stables d'agents actifs contenant des dérivés glucosiques tensiosactifs et d'acide salicylique

(30) Priorität: 29.04.1999 DE 19919481
(43) Veröffentlichungstag der Anmeldung: 02.11.2000
(60) Teilanmeldung: 07003633.0
(73) Patentinhaber: Beiersdorf AG, 20253 Hamburg (DE)
(72) Erfinder: Bormann, Angelika, 22041 Hamburg (DE); Nielsen, Jens, 24558 Henstedt-Ulzburg (DE); Herpens, Andreas, 21465 Reinbek (DE); Müller, Anja, 23843 Rümpel (DE); Kielholz, Jürgen, Dr., 22549 Hamburg (DE)

(56) Entgegenhaltungen:
- EP-A- 0 987 011
- DE-A- 19 645 319
- US-A- 5 411 742
- US-A- 5 531 993
- US-A- 5 863 544

## Beschreibung

Die vorliegende Erfindung betrifft Wirkstoffe und Zubereitungen, solche Wirkstoffe enthaltend, welche gegen unreine Haut und Akne wirksam sind.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen, also metastabile Zwei- oder Mehrphasensysteme bei welchen die einzelnen Phasen im flüssigen Zustande vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Um die Metastabilität von Emulsionen gewährleisten zu können, sind in der Regel grenzflächenaktive Substanzen, also Emulgatoren, nötig.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-Emulsionen gelegentlich zu Problemen führen, tritt dort aber bei weitem nicht so in den Vordergrund wie bei O/W-Systemen. Zwar läßt sich diesen oft durch geeignete Wahl des Emulgatorsystems in gewissem Maße Abhilfe schaffen, es treten dann aber ebensooft andere Nachteile auf.

Es ist andererseits oft wünschenswert, bestimmte Elektrolyte einzusetzen, um deren sonstige physikalische, chemische bzw. physiologische Eigenschaften nutzen zu können. So beschreiben die DE-OS 43 29 379 und die DE-OS 44 38 588 Zubereitungen gegen unreine Haut bzw. leichte Formen von Akne, die sich durch einen Gehalt an Wollwachssäurefraktionen auszeichnen,

Bei der unreinen Haut sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Zwar sind aus der DE 197 23 733 kosmetische und dermatologische Emulsionen bekannt mit mindestens einer wäßrigen Phase, enthaltend eine wirksame Menge einer oder mehrerer grenzflächenaktiven Substanzen, gewählt aus der Gruppe der Alkylglucoside, wobei mindestens eine der wäßrigen Phasen einen oder mehrere Elektrolyte gelöst enthält, wobei die lonenstärke der wäßrigen Phasen, in der der oder die Elektrolyte gelöst vorliegen, mindestens 0,075 mol/l beträgt.

Diese Schrift konnte jedoch nicht den Weg zur vorliegenden Erfindung bahnen.

Eine Aufgabe der vorliegenden Erfindung war es also, Lösungswege zu kosmetischen oder dermatologischen Zubereitungen gegen Akne, insbesondere Emulsionen, bevorzugt O/W-Emulsionen, aufzudecken, welche gegenüber erhöhten Elektrolytkonzentrationen - bzw. erhöhten lonenstärken - stabil sind.

Es hat sich überraschenderweise gezeigt, und darin liegt die Lösung der Aufgabe, daß die Verwendung kosmetischer und/oder dermatologischer Zubereitungen in Form von Emulsionen, enthaltend
a) eine wirksame Menge an Salicylsäure
   und
b) eine oder mehrere grenzflächenaktive Substanzen A, gewählt aus der Gruppe der Glucosederivate, welche sich durch die Strukturformel 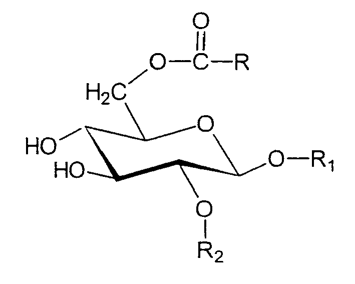 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,
(c) einer oder mehreren grenzflächenaktiven Substanzen B, gewählt aus der Gruppe der Substanzen der allgemeinen Strukturformel 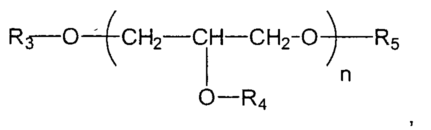 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt
   und
d) die Zubereitungen auf pH-Werte von 5,5 oder Kleiner abgepuffert sind.

Vorteilhaft wird R der grenzflächenaktiven Substanzen A aus der Gruppe der Glucosederivate gewählt aus der Gruppe der unverzweigten Alkylreste, wobei der Myristylrest, der Palmitylrest, der Stearylrest und der Eicosylrest bevorzugt werden.

R₁ der grenzflächenaktiven Substanzen A aus der Gruppe der Glucosederivate kann vorteilhaft ein Wasserstoffatom darstellen, wird aber bevorzugt aus der Gruppe Methyl-, Ethyl-, Propyl- und Isopropyl- gewählt.

R₂ der grenzflächenaktiven Substanzen A aus der Gruppe der Glucosederivate kann vorteilhaft ein Wasserstoffatom darstellen, kann aber ebenfalls vorteilhaft aus der Gruppe Myristoyl-, Palmitoyl-, Stearoyl- und Eicosoylgewählt werden.

R₃ der grenzflächenaktiven Substanzen B kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

R₄ der grenzflächenaktiven Substanzen B kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

R₅ der grenzflächenaktiven Substanzen B kann vorteilhaft ein Wasserstoffatom darstellen, kann aber ebenfalls vorteilhaft aus der Gruppe Myristoyl-, Palmitoyl-, Stearoyl- und Eicosoyl- gewählt werden.

Besonders vorteilhaft wird oder werden die grenzflächenaktiven Substanzen A aus der Gruppe Methylglucosemonostearat (Formel wie nachstehend)

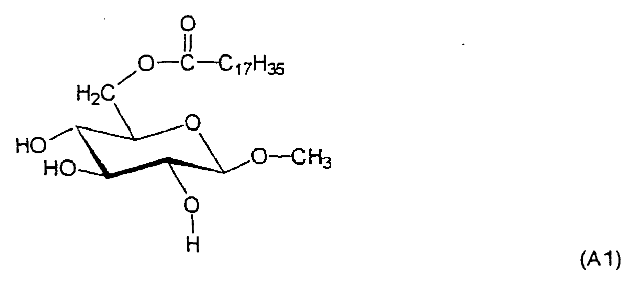

Methylglucosedistearat (Formel wie nachstehend) 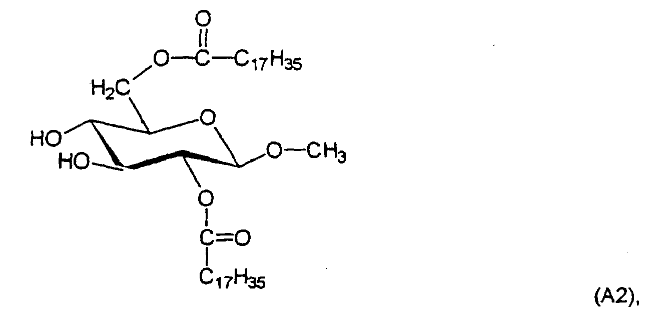 und beliebigen Gemischen daraus, beispielsweise ungefähr äquimolaren Mischungen daraus gewählt, welche auch als Methylglucosesesquistearat bezeichnet werden. Solches Methylglucosesesquistearat ist im Handel beispielsweise unter der Warenbezeichnung Tego® Care PS von der Gesellschaft Th.Goldschmidt KG erhältlich.

Die Gesamtmenge an einer oder mehreren erfindungsgemäß verwendeten grenzflächenaktiven Glucosederivaten in den fertigen kosmetischen oder dermatologischen Zubereitungen wird vorteilhaft aus dem Bereich von 0,1 - 25,0 Gew.-%, bevorzugt 0,5 - 15,0 Gew.-% gewählt, bezogen auf das Gesamtgewicht der Zubereitungen.

Angemerkt sei, daß glucosylierte Produkte in der Regel nicht reine "Monoglucoside" darstellen, sondern sich vielmehr, herstellungsbedingt, durch einen Glucosylierungsgrad DP auszeichnen, welcher am unveresterten Molekül nachfolgend dargestellt wird:

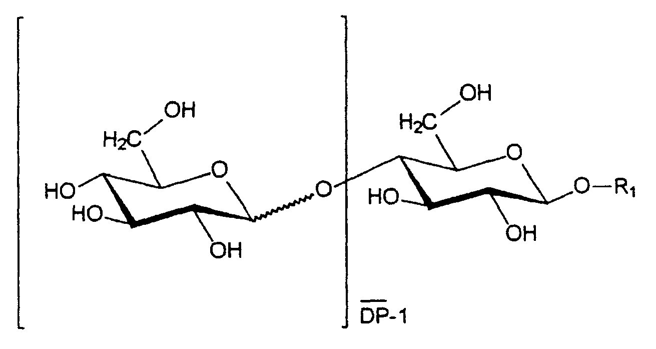

Übliche Glucosylierungsgrade werden gewählt aus dem Bereich von 1 - 2, bevorzugt von 1,1 - 1,5, insbesondere bevorzugt von ungefähr 1,3.

Besonders vorteilhaft wird oder werden die grenzflächenaktiven Substanzen B gewählt aus der Gruppe der Verbindungen, bei welcher n den Wert 3 annimmt und R₃, R₄ und R₅ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen:

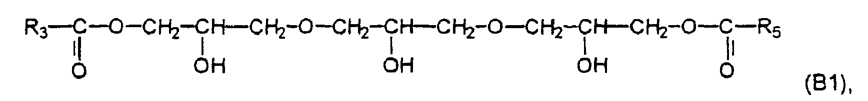

Erfindungsgemäß erhältliche Emulsionen sind nicht nur erstaunlich stabil, sie sind auch besser gegen unreine Haut und Akne wirksam als der Stand der Technik hätte erwarten lassen. Darüberhinaus zeichnen sie sich auch durch gesteigerte Hautverträglichkeit und bessere kosmetische Erscheinung aus.

Die Gesamtmenge an einer oder mehreren erfindungsgemäß verwendeten grenzflächenaktiven Substan-zen B in den fertigen kosmetischen oder dermatologischen Zubereitungen wird vorteilhaft aus dem Bereich von 0,1 - 25,0 Gew.-%, bevorzugt 0,5 - 15,0 Gew.-% gewählt, bezogen auf das Gesamtgewicht der Zubereitungen.

Die grenzflächenaktiven Substanzen A und B liegen vorteilhaft in Gewichtsverhältnissen zueinander von 20: 1 bis 1: 20, bevorzugt 10: 1 bis 1 zu 10, besonders bevorzugt 5: 1 bis 1: 5, ganz besonders bevorzugt 2: 1 bis 1: 2, vor.

Als erfindungsgemäß bevorzugt hat sich herausgestellt, ein ungefähr äquimolekulares Gemisch aus den Verbindungen A2 und B1, wobei in B1 die Reste R₃ und R₅ vorzugsweise beide einen Stearatrest bezeichnen, herausgestellt. Solche Emulgatorkombinationen sind, summarisch als "Polyglyceryl(3)-Methylglucosedistearat" (PGMS) bezeichnet, unter der Warenbezeichnung Tego Care® 450 von der Gesellschaft Th. Goldschmidt KG erhältlich.

Erfindungsgemäß können als günstige, aber dennoch fakultativ zu verwendende Antioxidantien alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-,γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Antioxidantien, Insektenrepellentien, Alkohol, Wasser, Salze, antimikrobiell, proteolytisch oder keratolytisch wirksame Substanzen usw.

Entsprechend können die erfindungsgemäßen Zusammensetzungen, je nach ihrem Aufbau, beispielsweise verwendet werden als Hautschutzcrème, Reinigungsmilch, Sonnenschutzlotion, Nährcrème, Tages- oder Nachtcröme usw. Es ist gegebenenfalls möglich und vorteilhaft, die erfindungsgemäßen Zusammensetzungen als Grundlage für pharmazeutische Formulierungen zu verwenden.

Erfindungsgemäß ist auch die Verwendung der Wirkstoffkombinationen in hautpflegenden kosmetischen und/oder dermatologischen Zubereitungen.

Zur Anwendung werden die erfindungsgemäßen kosmetischen und dermatologischen Zubereitungen in der für Kosmetika üblichen Weise auf die Haut in ausreichender Menge aufgebracht.

Erfindungsgemäße kosmetische und dermatologische Zubereitungen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, die erfindungsgemäßen Wirkstoffkombinationen in wäßrige Systeme bzw. Tensidzubereitungen zur Reinigung der Haut einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder demiatologische Zubereitung eine Lösung oder Lotion darstellt, können als Lösemittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykol-monoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäße Emulsionen, z.B. in Form einer Hautschutzcrème, einer Hautlotion, einer kosmetischen Milch, beispielsweise in Form einer Sonnenschutzcreme oder einer Sonnenschutzmilch, sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Erfindungsgemäße Zubereitungen können auch günstig als Gele vorliegen, die neben den erfindungsgemäßen Wirkstoffkombinationen und dafür üblicherweise verwendeten Lösemitteln, üblicherweise Alkohole niedriger C-Zahl, z.B. Ethanol, Isopropanol, 1,2-Propandiol, Glycerin und Wasser bzw. ein vorstehend genanntes Öl, dann noch organische Verdickungsmittel enthalten, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Siliciumdioxid und/oder Aluminiumsilikate wie beispielsweise Bentonite, oder ein Gemisch aus Polyethylenglykol und Polyethylenglykolstearat oder -distearat. Das oder die Verdickungsmittel sind im Gel z.B. in einer Menge zwischen 0,1 und 30 Gew.-%, bevorzugt zwischen 0,5 und 15 Gew.-%, enthalten.

Erfindungsgemäße feste Stifte enthalten z.B. natürliche oder synthetische Wachse, Fettalkohole oder Fettsäureester.

Als Treibmittel für erfindungsgemäße, aus Aerosolbehältern versprühbare kosmetische und/oder dermatofogische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können sie außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische und/oder dermatologische Zubereitungen zur Verfügung zu stellen, die die Haut vor dem gesamten Bereich der ultravioletten Strahlung schützen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, z.B. 3-(4-Methylbenzyliden)campher, 3-Benzyliden-campher;
- 4-Aminobenzoäsäure-Derivate, z.B. 4-(Dimethylamino)-benzoäsäure(2-ethylhexyl)ester, 4-(Dimethylamino)benzoäsäureamylester;
- Ester der Zimtsäure, z.B. 4-Methoxyzimtsäure(2-ethylhexyl)ester, 4-Methoxyzimtsäureisopentylester;
- Ester der Salicylsäure, z.B. Salicylsäure(2-ethylhexyl)ester, Salicylsäure(4-isopropylbenzyl)ester, Salicylsäurehomomenthylester,
- Derivate des Benzophenons, z.B. 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, z.B. 4-Methoxybenzalmalonsäuredi(2-ethylhexyl)ester, - 2,4,6-Trianilino-(p-carbo-2'-ethyl-1 '-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, wie z. B. 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Enthalten die erfindungsgemäßen Emulsionen UVA-Filtersubstanzen, können diese erfindungsgemäß vorteilhaft gewählt werden aus der Gruppe der Derivate des Dibenzoylmethans, z.B. 1-(4'-tert. Butylphenyl)-3-(4'-methoxyphenyl)propan-1, 3-dion und 1 -Phenyl3-(4'-isopropylphenyl)propan-1,3-dion.

Die erfindungsgemäßen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen bzw. Abwandlungen davon. Besonders bevorzugt handelt es sich um Pigmente auf der Basis von Titandioxid.

Bei erfindungsgemäßen kosmetischen und dermatologischen Zubereitungen für die Verwendung an behaarter Haut handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und/oder dermatologischen Zubereitungen enthalten gegebenenfalls zusätzliche Wirkstoffe, Hilfs- und/oder Zusatzstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte sowie zusätzliche Substanzen zum Rückfetten der Haare bzw. der Kopfhaut.

Erfindungsgemäße wäßrige kosmetische Reinigungsmittel mit einem Gehalt an den erfindungsgemäßen Wirkstoffkombinationen bzw. für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Feifsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobemsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wir, so handelt es sich dabei z.B. um wäßrige oder wäßrig-alkoholische Lösungen, die gegebenenfalls oberflächenaktive Substanzen enthalten, bevorzugt nichtionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen und/oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie die erfindungsgemäßen Wirkstoffkombinationen. Die Menge der verwendeten Polymeren liegt z.B. zwischen 0,1 und 10 Gew.-%, bevorzugt zwischen 0,1 und 3 Gew.-%.

Erfindungsgemäße kosmetische Zubereitungen zur Behandlung und Pflege der behaarten Haut, die erfindungsgemäße Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische und/oder dermatologische Zubereitung eine Lotion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösemittel verwendet. Bei alkoholischen Lösemitteln kann Wasser ein weiterer Bestandteil sein.

Erfindungsgemäße Emulsionen zur Nagelpflege enthalten z.B. die genannten Fette, Öle, Wachse und andere Fettkörper, sowie Wasser und einen Emulgator, wie er üblicherweise für einen solchen Typ der Formulierung verwendet wird.

Erfindungsgemäße Gele zur Pflege und/oder Wiederherstellung der Nägel enthalten üblicherweise Alkohole niedriger C-Zahl, z.B. Ethanol, Isopropanol, 1,2-Propandiol, Glycerin und Wasser bzw. ein vorstehend genanntes ÖI in Gegenwart eines Verdikkungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Ansonsten gelten für diese Gruppe an kosmetischen und/oder dermatologischen Zubereitungen die üblichen Anforderungen, die der Fachmann an solche Zubereitungen und deren Inhaltsstoffe stellt.

Die folgenden Beispiele wollen die vorliegende Erfindung erläutern, ohne daß eine Beschränkung auf den Gehalt der Beispiele beabsichtigt ist. Die Mengenangaben bedeuten stets, sofern nichts Anderes angegeben ist, Gewichts-%, bezogen auf das Gesamtgewicht der jeweiligen Zubereitungen.

### Beispiel 1

| | % |
|---|---|
| Polyglyceryl-3-methylglucose distearat | 4% |
| Glycerin | 5,000 |
| Sorbitandistearat | 3,000 |
| Cetylstearylalkohol | 2,000 |
| Salicylsäure | 1,500 |
| PEG-150-distearat | 0,500 |
| Octylmethocycinnamat | 4,000 |
| Xanthangummi | 0,500 |
| Natriumhydroxid | 0,350 |
| Titandioxid | 0,250 |
| Konservierungsmittel, Farbstoffe, Parfum | q.s. |
| Wasser | ad 100,00 |

## Patentansprüche

1. Emulsionen, enthaltend
a) eine wirksame Menge an Salicylsäure
und
b) eine oder mehrere grenzflächenaktive Substanzen A, gewählt aus der Gruppe der Glucosederivate, welche sich durch die Strukturformel 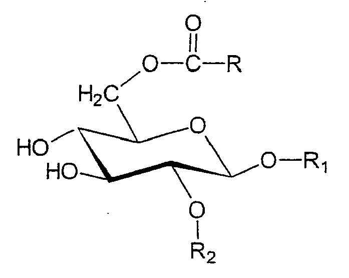 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,
(c) einer oder mehreren grenzflächenaktiven Substanzen B, gewählt aus der Gruppe der Substanzen der allgemeinen Strukturformel 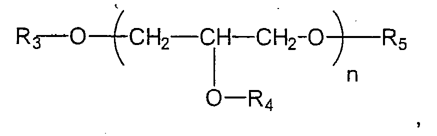 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt. und
d) die Zubereitungen auf pH-Werte von 5,5 oder Kleiner abgepuffert sind.

2. Emulsion nach Anspruch 1, **dadurch gekennzeichnet, daß** die grenzflächenaktiven Substanzen A aus der Gruppe Methylglucosemonostearat (A1) Methylglucosedistearat (A2), und beliebigen Gemischen daraus gewählt werden,

3. Emulsion nach Anspruch 1 oder 2, **dadurch gekennzeichnet, daß** die grenzflächenaktiven Substanzen B gewählt wird aus der Gruppe der Verbindungen, bei welcher n den Wert 3 annimmt und R₃, R₄ und R₅ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen:

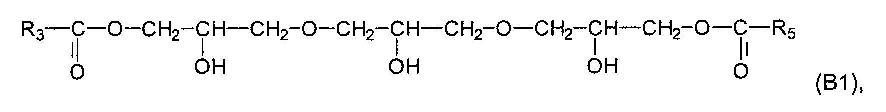

4. Emulsion nach einem der vorstehenden Ansprüche, **dadurch gekennzeichnet, daß** die Gesamtmenge an einer oder mehreren erfindungsgemäß verwendeten grenzflächenaktiven Substanzen A in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

5. Emulsion nach einem der vorstehenden Ansprüche, **dadurch gekennzeichnet, daß** die Gesamtmenge an einer oder mehreren erfindungsgemäß verwendeten grenzflächenaktiven Substanzen B in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

6. Emulsion nach einem der vorstehenden Ansprüche, **dadurch gekennzeichnet, daß** die grenzflächenaktiven Substanzen A und B in Gewichtsverhältnissen zueinander von 20 : 1 bis 1 : 20, bevorzugt 10: 1 bis 1 zu 10, besonders bevorzugt 5 : 1 bis 1 : 5, ganz besonders bevorzugt 2 : 1 bis 1 : 2, vorliegen.

7. Verwendung
a) einer wirksame Mengen an Salicylsäure
b) einer oder mehrerer grenzflächenaktiven Substanzen A, gewählt aus der Gruppe der Glucosederivate, welche sich durch die Strukturformel 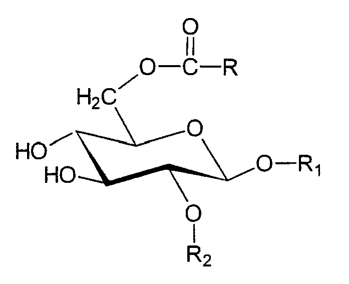 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,
(c) einer oder mehreren grenzflächenaktiven Substanzen B, gewählt aus der Gruppe der Substanzen der allgemeinen Strukturformel 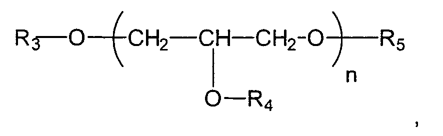 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt, und d) die Zubereitungen auf pH-Werte von 5,5 oder Kleiner abgepuffert sind.
zur Herstellung einer therapeutischen Zubereitung gegen Akne.

8. Verwendung nach Anspruch 6, **dadurch gekennzeichnet, daß** die Gesamtmenge an einer oder mehreren erfindungsgemäß verwendeten grenzflächenaktiven Substanzen A in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

9. Verwendung nach Anspruch 6 oder 7, **dadurch gekennzeichnet, daß** die Gesamtmenge an einer oder mehreren erfindungsgemäß verwendeten grenzflächenaktiven Substanzen B in den fertigen kosmetischen oder dermatologischen Zubereitungen aus dem Bereich von 0,1 - 25,0 Gew.-%, bevorzugt 0,5 - 15,0 Gew.-% gewählt wird, bezogen auf das Gesamtgewicht der Zubereitungen.

10. Verwendung nach Anspruch 6, 7 oder 8, **dadurch gekennzeichnet, daß** die grenzflächenaktiven Substanzen A und B in Gewichtsverhältnissen zueinander von 20 : 1 bis 1 : 20, bevorzugt 10 : 1 bis 1 zu 10, besonders bevorzugt 5 : 1 bis 1 : 5, ganz besonders bevorzugt 2 : 1 bis 1 : 2, vorliegen.

11. Kosmetische Verwendung einer Emulsion nach einem der Ansprüche 1- 5 gegen unreine Haut.

## Claims

1. Emulsions, comprising
(a) an effective amount of salicylic acid
and
(b) one or more interface-active substances A, chosen from the group of glucose derivatives, which are **characterized by** the structural formula 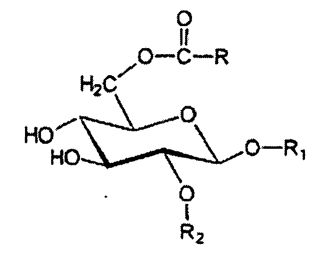 where R is a branched or unbranched alkyl radical having from 1 to 24 carbon atoms, where R1 is either a hydrogen atom or a branched or unbranched alkyl radical having from 1 to 24 carbon atoms, and where R2 is either a hydrogen atom or a branched or unbranched acyl radical having from 1 to 24 carbon atoms, comprising
(c) one or more interface-active substances B, chosen from the group of substances of the general structural formula 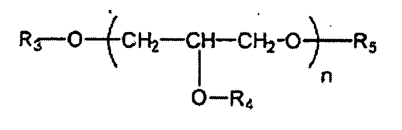 where R3, R4 and R5, independently of one another, are chosen from the group which consists of: H, branched or unbranched, saturated or unsaturated fatty acid radicals having from 8 to 24 carbon atoms, in which up to three aliphatic hydrogen atoms can be substituted by hydroxyl groups, and n is a number from 2 to 8,
and
(d) the preparations are buffered to a pH value of 5.5 or lower.

2. Emulsions corresponding to claim 1 wherein the interface-active substances A are chosen from the group consisting of methylglucose monostearate (A1), methylglucose distearate (A2) and any mixtures thereof

3. Emulsions corresponding to claim 1 or 2 wherein the interface-active substances B are chosen from the group of compounds in which n assumes the value 3, and R3, R4 and R5, independently of one another, are chosen from the group which consists of: H, branched or unbranched, saturated or unsaturated fatty acid radicals having from 14 to 20 carbon atoms, in particular the structures listed below:

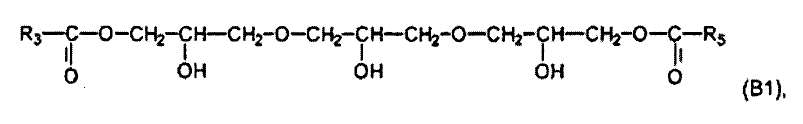

4. Emulsions corresponding to one of the above claims wherein the total amount of one or more interface-active substances A used according to the invention in the finished cosmetic or dermatological preparations is advantageously chosen from the range 0.1-25.0% by weight, preferably 0.5-15.0% by weight, based on the total weight of the preparations.

5. Emulsions corresponding to one of the above claims wherein the total amount of one or more interface-active substances B used according to the invention in the finished cosmetic or dermatological preparations is advantageously chosen from the range 0.1-25.0% by weight, preferably 0.5-15.0% by weight, based on the total weight of the preparations.

6. Emulsions corresponding to one of the above claims wherein the interface-active substances A and B are advantageously present in weight ratios to one another of from 20:1 to 1:20, preferably from 10:1 to 1:10, particularly preferably from 5:1 to 1:5, very particularly preferably from 2:1 to 1:2

7. Use
(a) of an effective amount of salicylic acid
(b) of one or more interface-active substances A, chosen form the group of glucose derivatives which are **characterized by** the structural formula 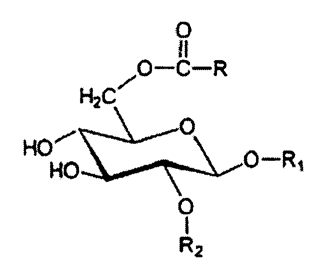 where R is a branched or unbranched alkyl radical having from 1 to 24 carbon atoms, where R1 is either a hydrogen atom or a branched or unbranched alkyl radical having from 1 to 24 carbon atoms, and where R2 is either a hydrogen atom or a branched or unbranched acyl radical having from 1 to 24 carbon atoms, comprising
(c) one or more interface-active substances B, chosen from the group of substances of the general structural formula 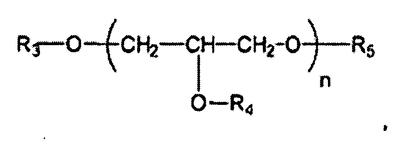 where R3, R4 and R5, independently of one another, are chosen from the group which consists of: H, branched or unbranched, saturated or unsaturated fatty acid radicals having from 8 to 24 carbon atoms, in which up to three aliphatic hydrogen atoms can be substituted by hydroxyl groups, and n is a number from 2 to 8, for the production of a therapeutic preparation against acne.
and
(d) the preparations are buffered to a pH value of 5.5 or lower.

8. Use corresponding to claim 6 wherein the total amount of one or more interface-active substances A used according to the invention in the finished cosmetic or dermatological preparations is advantageously chosen from the range 0.1-25.0% by weight, preferably 0.5-15.0% by weight, based on the total weight of the preparations.

9. Use corresponding to claim 6 or 7 wherein the total amount of one or more interface-active substances B used according to the invention in the finished cosmetic or dermatological preparations is advantageously chosen from the range 0.1-25.0% by weight, preferably 0.5-15.0% by weight, based on the total weight of the preparations.

10. Use corresponding to claim 6, 7 or 8 wherein the interface-active substances A and B are advantageously present in weight ratios to one another of from 20:1 to 1:20, preferably from 10:1 to 1:10, particularly preferably from 5:1 to 1:5, very particularly preferably from 2:1 to 1:2.

11. Cosmetic use of an emulsion corresponding to one of claims 1 -5 against blemished skin.

## Revendications

1. Emulsions contenant
a) une quantité active d'acides salicyliques
et
b) une ou plusieurs substances tensioactives A, sélectionnées dans le groupe des dérivés de glucose, lesquels se caractérisent par la formule de structure 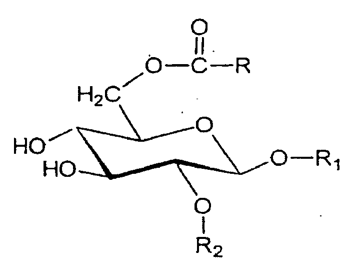 où R présente un reste d'alkyl ramifié ou non ramifié avec 1 à 24 atomes
De carbone, où R₁ présente un atome d'hydrogène ou un reste d'alkyl ramifié ou non avec 1 à 24 atomes de carbone et où R₂ présente un atome d'hydrogène ou un reste d'alkyl ramifié ou non avec 1 à 24 atomes de carbone, contenant
c) une ou plusieurs substances tensioactives B, sélectionnées dans le groupe des substances de la formule générale de structure 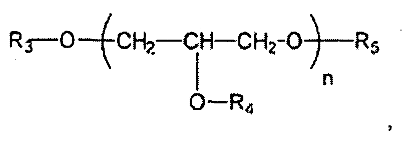 Où R3, R4, R5 sélectionnés dans le groupe indépendamment l'un de l'autre, lequel comprend H restes d'acides gras ramifiés ou non, saturés ou non saturés avec 8 à 24 atomes de carbone dans le cas desquels jusqu'à trois atomes d'hydrogène aliphatiques peuvent être substitués par des groupes d'hydroxydes et n présentant un nombre de 2 à 8 et
d) les préparations qui sont tamponnées sur l'indice pH 5,5, ou sur un indice inférieur

2. Une émulsion selon la revendication 1 **caractérisée par le fait que** les substances A tensioactives sont sélectionnées dans le groupe méthylglucosemonostéarate (A1), méthylglucosedistéarate (A2) et de mélanges quelconques.

3. Une émulsion selon la revendication 1 ou 2, **caractérisée par le fait que** les substances B tensioactives sont sélectionnées dans le groupe des combinaisons, pour lesquelles n prend la valeur 3 et R₃, R₄ et R₅ sont sélectionnées indépendamment l'une de l'autre à partir du groupe, lequel comprend des restes d'acides gras ramifiés ou non ramifiés, saturés ou non saturés avec 14 à 20 atomes de carbone, et surtout les structures présentées comme suit :

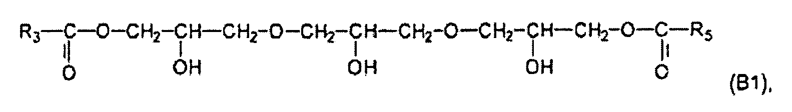

4. Une émulsion selon une des revendications précédentes, **caractérisées par le fait que** la quantité totale d'une ou de plusieurs des substances A tensioactives utilisées dans les préparations cosmétiques ou dermatologiques conformément à l'invention est sélectionnée dans le domaine allant de 0,1 - 25,0 en pourcentage du poids, de préférence 0,5 - 15,0 , par rapport au poids total des préparations.

5. Une émulsion selon une des revendications précédentes, **caractérisées par le fait que** la quantité totale d'une ou de plusieurs des substances B tensioactives utilisées dans les préparations cosmétiques ou dermatologiques conformément à l'invention est sélectionnée dans le domaine allant de 0,1 - 25,0 en pourcentage du poids, de préférence 0,5 - 15,0 , par rapport au poids total des préparations.

6. Une émulsion selon une des revendications précédentes, **caractérisées par le fait que** les substances tensioactives A et B existent dans des rapports pondéraux de l'un à l'autre de 20 : 1 à 1 :20, de préférence 10 :1 1 à 1 par rapport à 10 et de préférence particulière 5 : 1 à 1 :5, de préférence toute particulière 2 : 1 à 1 : 2.

7. Utilisation
a) d'une quantité active en acides salicyliques
b) d'une ou plusieurs substances A tensioactives, sélectionnées à partir du groupe des dérivés de glucose, lesquels sont **caractérisés par** la formule de structure , 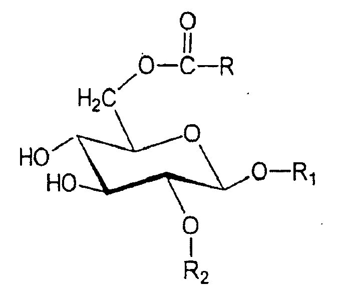 où R présente un reste d'alkyl avec 1 à 24 atomes de carbone, où R₁ présente un atome d'hydrogène ou un reste d'alkyl ramifié ou non ramifié avec 1 à 24 atomes de carbone et où R₂ présente un atome d'hydrogène ou un reste d'alkyl avec 1 à 24 atomes de carbone
c) une ou plusieurs substances tensioactives B sélectionnées à partir du groupe des substances de formule générale 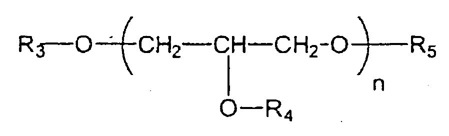 où R3, R4, R5 sont sélectionnés séparément dans le groupe lequel contient : H reste d'acides gras ramifiés ou non ramifiés, saturés ou non avec 8 à 24 atomes de carbone, pour lesquels jusqu'à 3 atomes d'hydrogène aliphatiques peuvent être substitués par des groupemest hydroxyles et n présentant un nombre de 2 à 8 et est utilisé pour la préparation d'une solution thérapeutique contre l'acné.
d) les préparations sont tamponées sur l'indice pH 5,5 ou sur un indice inférieur.

8. Utilisation selon la revendication 6, **caractérisée par le fait que** la quantité totale d'une ou de plusieurs substances tensioactives A utilisées conformément à l'invention dans les préparations cosmétiques ou dermatologiques est sélectionnée dans le domaine de 0,1 - 25,0 en pourcentage du poids de préférence 0,5 - 15,0 , par rapport au poids total des préparations.

9. Utilisation selon la revendication 6 ou 7, **caractérisée par le fait que** la quantité totale d'une ou de plusieurs substances tensioactives B utilisées conformément à l'invention dans les préparations cosmétiques ou dermatologiques est sélectionnée dans le domaine de 0,1 - 25,0 en pourcentage du poids de préférence 0,5 - 15,0 , par rapport au poids total des préparations.

10. Utilisation selon la revendication 6,7 ou 8, **caractérisée par le fait que** les substances tensioactives A et B existent dans des rapports pondéraux de l'un à l'autre de 20 : 1 à 1 :20, de préférence 10 : 1 à 1 par rapport à 10, de préférence particulière 5 : 1 à 1 : de préférence toute particulière 2 : 1 à 1 : 2.

11. l'utilisation cosmétique d'une émulsion selon une des exigences 1 à 5 contre les peaux malades.
